# EUROPÄISCHE PATENTSCHRIFT

(11) **EP 4 452 343 B1**
(45) Veröffentlichungstag und Bekanntmachung des Hinweises auf die Patenterteilung: **12.11.2025**
(21) Anmeldenummer: 22843716.6
(22) Anmeldetag: 21.12.2022
(51) Int. Cl.: A61L 26/00

(54) **WUNDMITTEL, WUNDGEL UND KOSMETIKUM**
WOUND DRESSING, WOUND GEL AND COSMETIC
PANSEMENTS, GEL POUR LES PLAIES ET PRODUIT COSMÉTIQUE

(30) Priorität: 23.12.2021 DE 102021215013
(43) Veröffentlichungstag der Anmeldung: 30.10.2024
(73) Patentinhaber: PolyMedics Innovations GmbH, 73230 Kirchheim unter Teck (DE)
(72) Erfinder: PLANCK, Heinrich, 72622 Nürtingen (DE); MÜLLER, Erhard, 70565 Stuttgart (DE); REIMER, Svenja, 72631 Aichtal (DE); PLANCK, Christian, 73230 Kirchheim (DE); ERKEN, Peter, 72070 Tübingen (DE)
(74) Vertreter: Kohler Schmid Möbus Patentanwälte
(86) Internationale Anmeldenummer: PCT/EP2022/087354
(87) Internationale Veröffentlichungsnummer: WO 2023/118370

(56) Entgegenhaltungen:
- DE-A1- 102005 042 707
- DE-A1- 102014 211 356
- US-A1- 2009 004 271

## Beschreibung

Die Erfindung betrifft ein Wundmittel, ein Wundgel sowie ein Kosmetikum, insbesondere zur Hautpflege.

Die DE 10 2014 211 356 A1 offenbart ein Wundkontaktmaterial, das bei Kontakt mit einer Wunde Milchsäure und/oder Laktat freisetzen kann.

Aus der US 2009/004271 A1 ist ein Wundbehandlungsmaterial bekannt, das zerkleinerten Schaum zur Wundbehandlung aufweist. Der zerkleinerte Schaum enthält wenigstens ein biokompatibles Polymer.

Bei größeren äußeren Gewebedefekten besteht häufig ein stark erhöhtes Infektionsrisiko bzw. ist eine bakterielle (Misch-)Infektion des verletzten Gewebebereichs bereits häufig gegeben. Derlei Wunden heilen häufig nur mit ausgeprägten Vernarbungen ab. Die Gewebedefekte müssen in der klinischen Praxis oftmals mit einem Hautersatz in Form eines synthetischen oder biologischen Flächenmaterials gedeckt werden, um die sekundäre Wundheilung zu unterstützen und Infektionen entgegenzuwirken. Gerade bei tiefen oder unregelmäßigen Gewebedefekten ist der die Wundheilung unterstützende Effekt solcher Flächenmaterialien allerdings mitunter recht begrenzt. So kann es selbst bei Einsatz von Vakuum-Schaumsystemen und granulationsfördernden Kunsthautdeckungen in tiefer gelegenen Gewebeschichten zu einer nur unbefriedigenden und allzu verzögerten Wundheilung kommen, die wiederholte chirurgische Interventionen und Wundanfrischungen erforderlich machen.

Es ist deshalb die Aufgabe der Erfindung, ein synthetisches Wundmittel sowie ein synthetisches Wundgel anzugeben, die sowohl zur großflächigen Anwendung geeignet sind und gerade auch bei tieferen Gewebedefekten die Wundheilung unterstützen. Darüber hinaus ist es die Aufgabe der Erfindung, ein synthetisches Kosmetikum mit einer hautneutralen entzündungshemmenden Wirkung anzugeben, das insbesondere zur nachsorgenden Pflege abgeheilter Hautwunden geeignet ist.

Die das Wundmittel betreffende Aufgabe wird durch ein Wundmittel mit den in Anspruch 1 angegebenen Merkmalen gelöst. Das erfindungsgemäße Wundgel bzw. Kosmetikum weist die in Anspruch 8 angegebenen Merkmale auf. Bevorzugte Weiterbildungen der Erfindung sind in den Unteransprüchen und der Beschreibung angegeben.

Das erfindungsgemäße Wundmittel liegt in Form eines Schüttguts umfassend resorbierbare Partikel mit einer maximalen Abmessung, d. h. einer maximalen Längenerstreckung, von 5 Millimetern vor. Die Partikel weisen jeweils eine Porosität von jeweils über 80% auf, wobei die Partikel jeweils ein Co- und/oder Terpolymer auf der Basis von Polyhydroxycarbonsäuren, nämlich der Monomere Lactid, Trimethylencarbonat (TMC), Caprolacton und/oder 1,4-Dioxan-2-on oder Polyhydroxybutyrat (PHB), umfassen. Besonders bevorzugt umfassen die Partikel jeweils ein Copolymer auf Basis von Lactid und TMC oder auf Basis von Lactid und Caprolacton oder ein Terpolymer mit Lactid und TMC oder mit Lactid und Caprolacton und zusätzlich jeweils 1,4-Dioxan-2-on oder Polyhydroxybutyrat (PHB).

Das synthetische Wundmittel kann aufgrund seiner Partikelstruktur als Schüttgut selbst in tiefergelegene und für Flächenmaterialien nur bedingt oder nicht zugängliche Wundbereiche eingebracht, beispielsweise eingeblasen werden und so dort seine die Wundheilung unterstützende Wirkung entfalten. Das Schüttgut ist sowohl für größere als auch für kleinere Gewebedefekte geeignet, und kann besonders einfach in tiefere Wundabschnitte eingebracht werden. Insgesamt kann ein Gewebedefekt so vollständig mit dem Wundmittel aus- bzw. aufgefüllt werden. Durch die enorme Porosität der Partikel kann das Wundmittel Wundsekrete, entzündlich bedingte Exsudate sowie Blut wirkungsvoll ab- und adsorbieren. Bei der Resorption des Wundmittels freigesetztes Laktat ermöglicht, den pH-Wert im Bereich des gesamten Gewebedefekts, d. h. auch am schwer zugänglichen Wundgrund, im (leicht) sauren Bereich zu halten, wodurch eine keimabtötende Wirkung vermittelt und die Wundheilung insgesamt begünstigt wird. Insgesamt kann das Wundmittel eine antiinfektiöse sowie schmerzreduzierende Wirkung entfalten, wobei eine vollständige hydrolytische und enzymatische Abbaubarkeit gegeben ist. Angesichts der vollständigen Resorbierbarkeit des Wundmittels erübrigt sich ein späteres Entfernen des Wundmittels vollständig. Auch dies ist für das Bilden von Granulationsgewebe im Rahmen des sekundären Wundverschlusses günstig. Das Wundmittel ist ohne Weiteres steril bereitstellbar. Nach der Erfindung können die Schüttgut-Partikel insbesondere eine maximale Abmessung von 2 mm aufweisen. Dadurch kann das Wundmittel auch in feine Wunden mit nur geringen Abmessungen eingebracht werden.

Nach einer besonders bevorzugten Weiterbildung der Erfindung können die Schüttgut-Partikel des Wundmittels zumindest teilweise oder insgesamt pulverförmig, d. h. als Mikropartikel, ausgebildet sein. Die Partikel weisen in diesem Fall eine maximale Abmessung von 700 µm oder 150 µm, ganz besonders bevorzugt von nur 100 µm, auf. Dadurch kann das pulverförmige Wundmittel selbst in kleinste Wundvertiefungen eingebracht werden und dort seine Wirkung entfalten. Das Wundmittel ist ohne Weiteres steril bereitstellbar. Zudem kann das Raumvolumen einer Wunde mit einer solchen Korngröße der Partikel hohlraumfrei oder im Wesentlichen hohlraumfrei, d. h. ohne größere Hohlräume zwischen den Partikeln des Wundmittels, ausgefüllt werden. In der Folge kann im Defektvolumen des geschädigten Gewebes so insgesamt ein die Wundheilung begünstigendes Mikromilieu eingestellt und einer unerwünschten Infektion entgegengewirkt werden. Das pulverförmige Schüttgut kann insbesondere bei einer maximalen Abmessung der Partikel von 250 µm bzw. 100 µm in äußere Wunden bzw. - zur Adhäsionsprophylaxe - auch intraperitoneal eingeblasen werden.

Die maximale Abmessung der Schüttgut-Partikel kann bei größeren Partikeln im Wege der lichtmikroskopischen Vermessung der einzelnen Partikel bestimmt werden. Weist das pulverförmige Schüttgut Partikel mit einer maximalen Abmessung von 250 µm oder sogar nur 100 µm auf, so kann die jeweilige maximale Abmessung der Partikel des Schüttguts auch im Wege der Rasterelektronenmikroskopie experimentell bestimmt werden.

Die lichtmikroskopische als auch die rasterelektronenmikroskopische Bestimmung der jeweiligen maximalen Abmessung der Partikel kann insbesondere mittels einer computerbasierten optischen Analysesoftware teil- oder vollautomatisiert erfolgen. Dies ist für die zügige und fehlerarme Prüfung der Größe der Partikel von Vorteil.

Die Partikel des Schüttguts/Wundmittels weisen erfindungsgemäß mehrheitlich oder allesamt eine spratzige, d. h. unregelmäßige, Grundform auf. Die Partikel sind also nicht spährisch oder gerundet ausgeführt. Dadurch kann das Wundmittel nach Applikation in einer Wunde eine mechanisch stabilisierende Funktion für das die Wunde umgebende Gewebe aufweisen. Auch dadurch kann die Wundheilung bis zur vollständigen Resorption des Wundmittels weiter begünstigt werden.

Die Partikel des Wundmittels weisen nach der Erfindung bevorzugt eine mittlere Porengröße im Bereich von 0,1 µm bis 50 µm, insbesondere im Bereich zwischen 0,5 µm bis 30 µm, auf. Die mittlere Porengröße in Verbindung mit der außerordentlich großen Porosität der Partikel von über 90 % gewährleistet ein besonders zuverlässiges Absorbieren von Flüssigkeiten. Die innere Oberfläche der Partikel kann dadurch zügig und umfassend mit Flüssigkeit benetzt und die Partikel nicht zuletzt deshalb zuverlässig resorbiert werden.

Das Wundmittel weist in Form des vorstehend erläuterten pulverförmigen Schüttguts, d. h. in Form des Mikropulvers, vorzugsweise eine Schüttdichte, d. h. ein Verhältnis der Masse der Schüttung zum eingenommenen Schüttvolumen, zwischen 0,08 g/ml und 0,25 g/ml, insbesondere zwischen 0,12 und 0,14 g/ml gemäß der in der DIN EN ISO 60:2000-01 definierten Messmethodik auf.

Weisen die Partikel im Sinne von Micrografts eine maximale Abmessung zwischen 1 Millimeter und 5 Millimeter auf, so kann das Schüttgut bzw. Wundmittel vorzugsweise ein Volumengewicht zwischen 0,03 g/ml und 0,06 g/ml, insbesondere von ungefähr 0,04 g/ml aufweisen.

Die Partikel des schüttgutförmigen Wundmittels weisen nach der Erfindung eine bimodale Porenstruktur bzw. Verteilung ihrer Porengröße mit einer ersten Porengröße im Bereich von 0,1 µm bis 50 µm, insbesondere im Bereich 0,5 bis 30 µm, und mit einer zweiten Porengröße im Bereich von 80 µm bis 600 µm, insbesondere im Bereich von 100 µm bis 500 µm, auf. Dadurch kann bei Wundapplikation des Wundmittels eine besonders schnelle und wirksame Flüssigkeitsaufnahme durch die Partikel erreicht werden. Eine solche bimodale Porenstruktur lässt sich einerseits durch den weiter unten beschriebenen Trocknungsprozess einer zur Herstellung der Partikel eingesetzten Polymerlösung sowie durch zusätzliches Versehen der Polymerlösung mit Zucker vorab des Beginns des Trocknungsprozesses und zeitversetztes Entfernen des Zuckers erreichen.

Die Partikel können nach der Erfindung eine Polyhydroxycarbonsäure, insbesondere ein Terpolymer aus 65 bis 90 Gew. % Lactid (insbesondere D,L-Lactid), 5 bis 20 Gew. % Trimethylencarbonat und 5 bis 20 Gew. % ε-Caprolacton umfassen. In dem Terpolymer können die Monomere Lactid, Trimethylencarbonat und ε-Caprolacton insbesondere im Bereich von 85/10/5 bis 70/20/10 Gew. % vorliegen oder Mischungen hiervon.

Das Material des Wundmittels weist nach der Erfindung eine Glasübergangstemperatur Tg zwischen 22° C und 45° C auf. Dadurch bleibt das Wundmittel bei den bei Transport und Lagerung üblichen Umgebungstemperaturen formstabil und zeigt keine unerwünschten Agglomerationen. Dadurch ist die Rieselfähigkeit des Wundmittels gewährleistet. Erst bei Applikation kann es durch die lokale Wärmeeinwirkung des jeweilig umliegenden (Wund-)Gewebes erweichen und sich so optimal an das (Wund-)Gewebe anlegen. Die Bestimmung der Glasübergangstemperatur des Materials des Wundmittels erfolgt in an sich bekannter Weise im Wege der Dynamischen Differenz-Thermoanalyse gemäß DIN EN ISO 11357-1.

Nach der Erfindung können die Partikel jeweils einen Gehalt an freien Monomeren von 0 bis 12 Gew-%, insbesondere 0,1 bis 12 Gew.-%, vorzugsweise 1 bis 10 Gew.-%, bezogen auf das Gewicht des Co- und/oder Terpolymers, aufweisen.

Nach einer bevorzugten Weiterbildung der Erfindung kann das Co- und/oder Terpolymer mit einem Füllmaterial, insbesondere einem synthetischen Polymer, vorzugsweise Polyvinylalkohol (PVA), dotiert sein. Die emulgierende Wirkung des PVA vereinfacht die Herstellung der Partikel.

Die Partikel des Schüttguts/Wundmittels weisen erfindungsgemäß mehrheitlich oder allesamt eine gespratzte Grundform auf. Dadurch können sich die Partikel des Wundmittels nach Applikation in einer Wunde ineinander verkrallen und sich so in ihrer Position und Lage bezüglich der Wundflächen - und mithin auch die Wundflächen selbst - relativ zueinander stabilisieren. Dies ist für die Wundheilung vorteilhaft.

Das Wundgel umfasst das vorstehend erläuterte Wundmittel und kann insbesondere als Wundauflage oder zur intraperitonealen Adhäsionsprophylaxe bei der medizinischen Behandlung von Mensch und/oder Tier eingesetzt werden. Das Wundgel kann hierzu in die Bauchhöhle eingespritzt oder eingesprüht werden.

Das Wundgel kann nach der Erfindung neben dem Wundmittel (und Wasser) einen oder mehrere weitere Arzneistoffe und/oder Zusatzstoffe umfassen. Durch geeignete Zusatzstoffe kann beispielsweise die Konsistenz, die Haltbarkeit, eine kühlende Wirkung des Wundgels usw. eingestellt werden. So kann das Wundgel insbesondere ein biokompatibles Verdickungsmittel wie etwa ein wasserlösliches und/oder quellendes nicht-ionisches Polymer, insbesondere ein Poloxamer, Polyvinylalkohol, Polyethylenglykol, Hyaluronsäure, Kollagen, Gelatine, Alginat oder ein Cellulosederivat wie Hydroxypropylmethylcellulose, oder einen Emulgator, etwa Polyglyceryl-2 Triisostearat, oder sonstige Träger wie Kochsalzlösung oder Vaseline, umfassen.

Das Wundmittel kann nach der Erfindung auch als Füll- bzw. Hilfs- oder Wirkstoff bei Kosmetika, insbesondere zur Hautpflege, eingesetzt werden.

Das erfindungsgemäße Wundmittel kann beispielsweise in der nachstehend angegebenen Weise erzeugt werden:
Zunächst wird in einem ersten Schritt eine Polymerlösung aus einem Co- und/oder Terpolymer auf der Basis der Monomere Lactid, Trimethylencarbonat, Caprolacton und/oder 1, 4-Dioxan-2-on bzw. Polyhydroxybutyrat sowie einem geeigneten Lösungsmittel, beispielsweise Dimethylsulfoxid (DMSO), hergestellt. Beispielsweise wird eine 10% ige Polymerlösung mit 4% PVA (Polyvinylalkohol) in Dimethylsulfoxid (DMSO) hergestellt.

Die Polymerlösung wird sodann auf einen, vorzugsweise flächigen, Träger aufgebracht. Dies kann beispielsweise unter Ablass der Polymerlösung, etwa über ein Sieb mit definierter Maschenweite von beispielhaft 0,042 mm, erfolgen. Der Träger kann beispielsweise eine Glasplatte sein. Die Polymerlösung kann auf dem Träger mittels eines Rakels bzw. mittels eines Filmziehgeräts zu einem Film ausgezogen und zusätzlich mit einem Überschuss an Zucker, vorzugsweise Saccharose, versehen werden.

Das Lösungsmittel wird in einem nachfolgenden Schritt durch Trocknen, insbesondere durch Gefriertrocknen, unter Bildung eines Polymerkuchens entfernt. Das Trocknen kann beispielsweise wie folgt erfolgen:
Zum Gefriertrocknen wird die Polymerlösung vorzugsweise in einem ersten Teilschritt zumindest 12 Stunden bei -60° C und einem Vakuum (d. h. bei einem subatmosphärischen Umgebungsdruck) von ≤ 0,5 mbar gehalten.

Im Bedarfsfall kann der Polymerkuchen nachfolgend in einem mit VE-Wasser (vollentsalztem Wasser) befüllten Bad bei Umwälzung und Temperierung (bei ~21°C) über einen Zeitraum von ca. 40 min - 70 min inkubiert und sodann von dem Träger vereinfacht abgelöst werden.

Der Polymerkuchen wird nachfolgend in bidestilliertes Wasser eingelegt und so über jeweils 2 Stunden gewaschen und nachfolgend über zumindest 12 Stunden bei einem Vakuum von ≤ 0,5 mbar und -60 °C gefriergetrocknet
In einem nachfolgendem fakultativen Schritt wird der Polymerkuchen 58 unter Reinraumbedingungen bei zirka + 21° C und 30 % rel. Luftfeuchte über weitere zumindest 12 Stunden getrocknet.

Nachfolgend wird der so erhaltene Polymerkuchen, insbesondere durch ein- oder mehrstufiges Schroten bzw. Mahlen oder durch andere bekannte Verfahren, zum Schüttgut bzw. zum Wundmittel mit der jeweilig vorgegebenen maximalen Abmessung d der Partikel zerkleinert.

So kann der Polymerkuchen beispielsweise auf ungefähr 2 x 2 cm große Polymerkuchenstücke vorzerkleinert und dabei vorzugsweise mit flüssigem Stickstoff gekühlt werden. Durch den flüssigen Stickstoff kann eine Vorversprödung des Polymerkuchens und damit eine verbesserte Verarbeitbarkeit erreicht werden. Diese Polymerkuchenstücke können sodann mitsamt einer zur Kühlung/Versprödung ausreichenden Menge flüssigen Stickstoffs mittels einer Cryomühle (z.B. mit 12-Zahn-Rotor bei 18.000 U/min und mit Ringsieb 1,0- 0,5 mm) weiter zum Wundmittel10/Schüttgut 12 zerkleinert werden. Als Cryomühle kann beispielsweise die Ultra-Zentrifugalmühle ZM 200 der Firma RETSCH GmbH, Retsch-Allee 1-5, 42781 Haan, Deutschland, eingesetzt werden. Im Schüttgut ggf. noch enthaltene zu große Partikel können, soweit erforderlich, durch ein fakultatives Vorsieben des Schüttguts (gemäß DIN 66165) und nachfolgend auf Basis einer optischen (lichtmikroskopische oder rasterelektronenmikroskopische) Größenbestimmung der Partikel des Schüttguts detektiert und aus dem Schüttgut entfernt werden.

Das so erhaltene Schüttgut/Wundmittel wird nachfolgend vorzugsweise für zumindest 12 Stunden bei einem atmosphärischem Umgebungsdruck von unter 2 mbar vakuumgetrocknet. Daudurch kann das Schüttgut mit einem für Verpackungs- Lagerungs- und Transsportzwecke geeigneten Trocknungsgrad bereitgestellt und so die Rieselfähigkeit des Wundmittels bei dessen Applikation gewährleistet werden.

Darüber hinaus ist das erfindungsgemäße Schüttgut als Matrix für ein Nährmedium zum Kultivieren tierischer oder pflanzlicher Zellen, d. h. für Zellkulturen, einsetzbar.

Nachfolgend ist die Erfindung anhand von in der Zeichnung wiedergegebenen Ausführungsbeispielen erläutert. Die gezeigten und beschriebenen Ausführungsformen sind nicht als abschließende Aufzählung zu verstehen, sondern haben für die Schilderung der Erfindung vielmehr beispielhaften Charakter.

In der Zeichnung zeigen:
- Fign. 1: REM-Aufnahmen eines Wundmittels in Form von Schuttgut aus resorbierbaren Partikeln, hier beispielhaft mit einer maximalen Partikelgröße zwischen 500 µm und 1 mm in der Übersicht (Fig. 1A) sowie in einem Detailausschnitt (Fig. 1B);
- Fign. 2: REM-Aufnahmen eines Wundmittels in Form von Schüttgut aus resorbierbaren Partikeln mit einer maximalen Partikelgröße zwischen 250 µm und 500 µm in der Übersicht (Fig. 2A) sowie in einem Detailausschnitt (Fig. 2B);
- Fign. 3: REM-Aufnahmen eines Wundmittels in Form von Schüttgut aus resorbierbaren Partikeln mit einer maximalen Partikelgröße zwischen 63 µm bis 250 µm in der Übersicht (Fig. 3A) sowie in einem Detailausschnitt (Fig. 3B);
- Fign. 4: REM-Aufnahmen ein einzelnes Partikel eines Wundmittels in Form von Schüttgut in einer Übersicht (Fig. 4A) und in einem stark vergrößerten Detailausschnitt (Fig. 4B);
- Fig. 5: eine REM-Aufnahme eines zu dem in Fign. 4 gezeigten Partikel ähnlichen Partikels eines Wundmittels mit Darstellung der planen bzw. im Wesentlichen planen Unterseite;
- Fig. 6: ein Blockschaltbild mit einzelnen Verfahrensschritten eines Herstellungsverfahrens eines Wundmittels in Form von Schüttgut; und
- Fig. 7: eine schematische Darstellung eines Wundgels bzw. eines Kosmetikums.

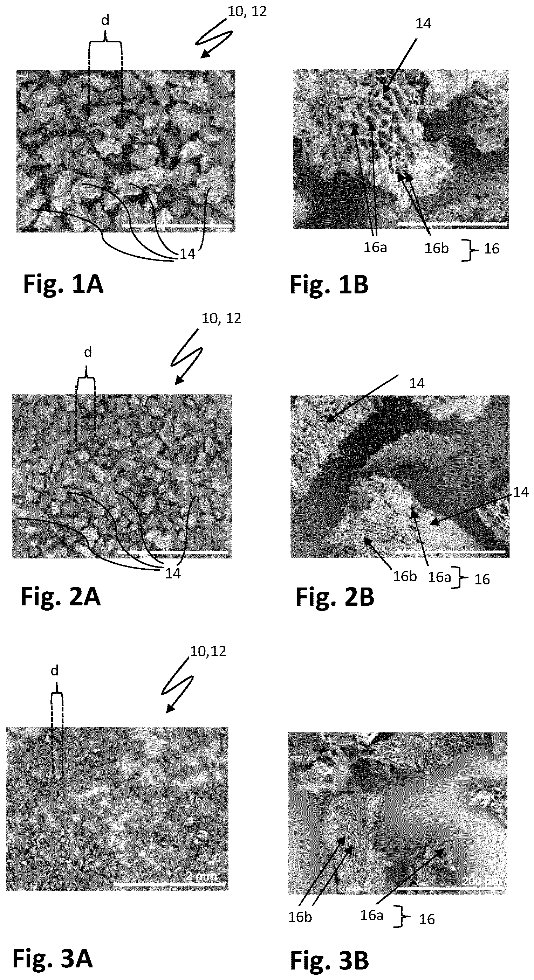

Fign. **1 bis 3** zeigen jeweils Rasterelektronenmikroskop (REM)-Aufnahmen von Wundmitteln **10.** Der in den Fign. 1A, 2A, 3A gezeigte und nicht näher bezeichnete Maßstab entspricht jeweils einheitlich einer Länge von 2 mm und in den Fign. 1B, 2B, 3B einer Länge von 200 µm.

Die gezeigten Wundmittel 10 sind jeweils in Form von Schüttgut **12** ausgeführt, das aus einer Vielzahl von Partikeln **14** besteht. So weist das Wundmittel 12 gemäß den Fign. 1 Partikel 14 mit einer maximalen Abmessung **d** (= maximale Längenerstreckung) zwischen 500 µm und 1 mm auf. Das in den Fign. 2 gezeigte Wundmittel 12 weist Partikel mit einer maximalen Abmessung zwischen 250 µm und 500 µm und das in den Fign. 3 gezeigte Wundmittel 12 weist Partikel 14 mit einer maximalen Abmessung zwischen 63 µm und 250 µm auf. Das schüttgutförmige Wundmittel 12 gemäß den Fign. 1A, 1B, 2A und 2B weist hier ein Volumengewicht zwischen 0,08 g/ml und 0,16 g/ml, insbesondere von zwischen 0,12 und 0,14 g/ml auf.

Die poröse Struktur der Partikel 14 ist gut zu erkennen. Die Porosität der Partikel 14 beträgt über 90 %. Die Poren 16 können nach größeren Primär- oder Makroporen **16a** und kleineren Sekundär- oder Mikroporen **16b** unterschieden werden. Die Partikel 14 weisen mehrheitlich oder allesamt jeweils eine spratzige, d. h. unregelmäßige, Grundform auf. Dadurch können sich die Partikel in der Wunde ineinander verhaken. So können die Wundränder einer Wunde durch das in die Wunde eingebrachte Wundpulver relativ zueinander mechanisch stabilisiert werden.

Die unterschiedlichen Feinheitsgrade des schüttgutförmigen Wundmittels 10 erlauben einen besonders breiten Einsatzbereich des Wundmittels 10. Je feiner, d. h. kleiner die Partikelgröße, desto besser ist das Wundmittel 10 für kleinere und kleinste Wunden anwendbar.

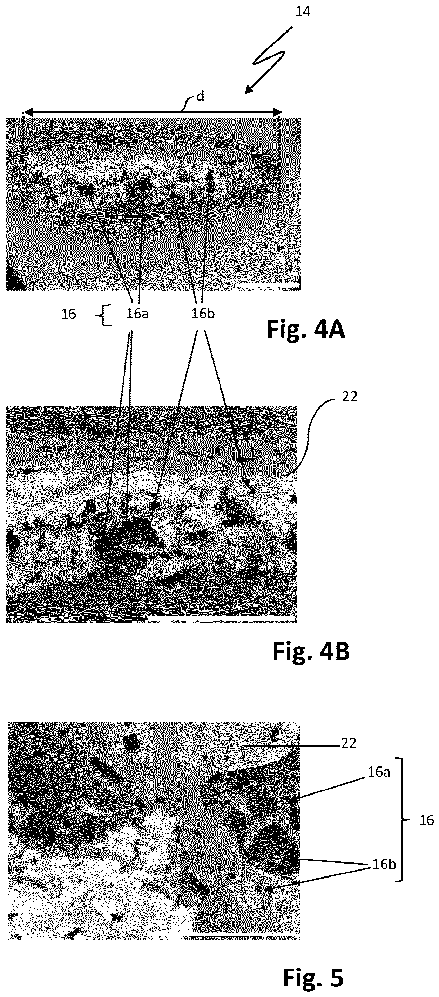

In den Fign. **4A** und **4B** sind REM-Aufnahmen eines einzelnen Partikels 14 eines Wundmittels 10 in unterschiedlichen Vergrößerungen gezeigt. Das Partikel 14 weist hier eine maximale Abmessung **d** von 3 Millimeter auf. Das Partikel 14 kann insoweit als ein Mikrograft bezeichnet werden und eignet sich ideal zur Behandlung großflächiger und unregelmäßig begrenzter Wunden. Die poröse Struktur des Partikels 14 ist in Fig. 4B gut zu erkennen. Die Poren 16 können auch hier nach größeren Primär- oder Makroporen 16a und kleineren Sekundär- oder Mikroporen 16b unterschieden werden. Das Partikel 14 weist ebenfalls eine gespratzte Grundform mit unregelmäßig begrenzten Seitenflanken **18** auf. Die spratzige Grundform des Partikels 14 ist auf die mechanische Zerkleinerung eines größeren Polymerkuchens während der Herstellung des Schüttguts bzw. des Partikels 14 zurückzuführen. Die Unterseite **22** des Partikels 14 zeigt eine überwiegend geschlossene und leicht wellige Topografie mit einzelnen Poren 16b. Die Unterseite **22** kann gemäß der in **Abb. 5** gezeigten REM-Aufnahme eines weiteren Partikels 14 relativ eben ausgeführt sein und nur wenige Makroporen 16a und zahlreiche Mikroporen 16b aufweisen. Der in den Fign. 4 und 5 gezeigte Maßstab entspricht jeweils 1 mm.

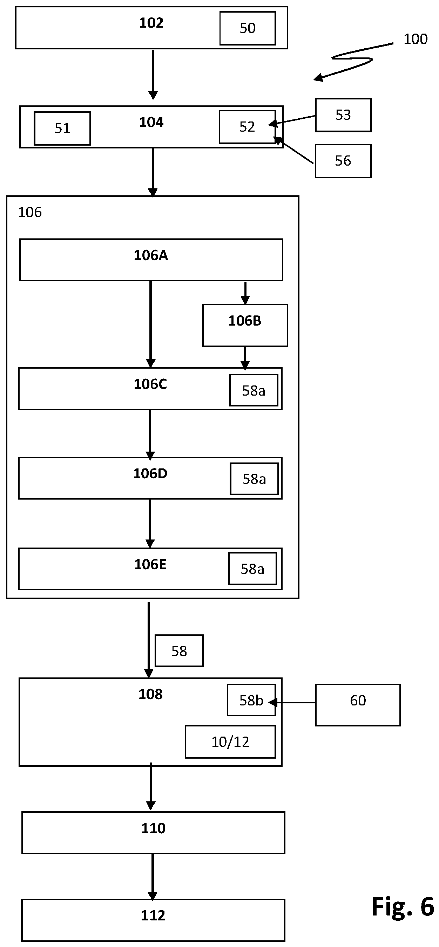

In **Fig. 6** ist ein Blockschaltbild mit einzelnen Verfahrensschritten eines Herstellungsverfahrens **100** für ein vorstehend im Zusammenhang mit den Fign. 1 bis 5 erläutertes Wundmittel 10 gezeigt.

In einem ersten Schritt **102** wird eine Polymerlösung **50** aus einem (selbstredend zuvor bereitgestellten) Co- und/oder Terpolymer auf der Basis der Monomere Lactid, Trimethylencarbonat, Caprolacton und/oder 1, 4-Dioxan-2-on bzw. Polyhydroxybutyrat (PHB) sowie einem geeigneten Lösungsmittel hergestellt. Beispielsweise kann eine 10% ige Polymerlösung 50 mit 4% PVA (Polyvinylalkohol) in DMSO hergestellt werden.

Diese Polymerlösung 50 wird in Schritt **104** beispielsweise unter Ablass der Polymerlösung (etwa über ein Sieb **51** mit beispielsweise 0,042 mm Maschenweite) auf einen Träger **52,** aufgebracht. Der Träger 52 kann insbesondere eine Glasplatte sein. Die Polymerlösung 50 kann auf dem Träger 52 mittels eines Rakels **54** bzw. eines Filmziehgeräts zu einem Film ausgezogen und zwecks Bildung der vorstehend erläuterten Makroporen 16a mit einem Überschuss an Zucker **56** versehen werden.

In einem weiteren Schritt **106** wird ein Polymerkuchen **58** erzeugt, indem das Lösungsmittel durch Trocknen, insbesondere durch Gefriertrocknen, entfernt wird.

Die Polymerlösung 50 wird dazu in einem Teilschritt **106A** über zumindest 12 Stunden bei -60° C und einem Vakuum von ≤ 0,5 mbar gefriergetrocknet.

Der so erzeugte Polymervorkuchen **58a** kann im fakultativen Zwischenschritt **106B** in einem mit VE-Wasser (vollentsaltem Wasser) befüllten Bad bei Umwälzung und Temperierung (~21°C) ca. 40 min - 70 min inkubiert und sodann von dem Träger vereinfacht abgelöst werden.

Der Polymervorkuchen 58a wird in nachfolgendem Schritt **106C** in bidestilliertes Wasser eingelegt und so über jeweils 2 Stunden gewaschen und nachfolgend in Schritt **106D** über zumindest 12 Stunden bei zumindest -60° Celsius und einem Vakuum von ≤ 0,5 mbar gefriergetrocknet.

In nachfolgendem Schritt **106E** kann der Polymervorkuchen 58a unter Reinraumbedingungen bei zirka + 21° C und 30 % rel. Luftfeuchte über weitere zumindest 12 Stunden getrocknet.

Der so erhaltene Polymerkuchen 58 wird in einem nachfolgenden Schritt **108** zum Wundmittel 10 in Form von Schüttgut 12 mit der jeweilig vorgegebenen maximalen Partikelgröße/Abmessung d (vgl. Fign. 1-4) der Partikel 14 mechanisch zerkleinert. Dies kann insbesondere durch ein- oder mehrstufiges Schroten bzw. Mahlen oder durch andere, dem Stand der Technik entsprechenden Techniken erfolgen.

So kann der Polymerkuchen 58 in Schritt 108 insbesondere auf ungefähr 2 x 2 cm große Polymerkuchenstücke **58b** vorzerkleinert und dabei mit flüssigem Stickstoff **60** gekühlt werden. Diese Polymerkuchenstücke **58a** können sodann mitsamt einer zur Kühlung ausreichenden Menge flüssigen Stickstoffs mittels einer Cryomühle (z.B. mit 12-Zahn-Rotor bei 18.000 U/min und mit Ringsieb 1,0- 0,5 mm) weiter zum Wundmittel10/Schüttgut 12 zerkleinert werden.

Das so erhaltene (pulverförmige) Wundmittel 10/Schüttgut 12 wird vorzugsweise in Schritt **110** für zumindest 12 Stunden bei einem atmosphärischem Umgebungsdruck von unter 2 mbar vakuumgetrocknet.

Im Wundmittel 10/Schüttgut 12 ggf. noch enthaltene zu große Partikel können, soweit erforderlich, vor oder nach Schritt 110 durch ein fakultatives Vorsieben des Schüttguts (gemäß DIN 66165) und auf Basis einer optischen (lichtmikroskopische oder rasterelektronenmikroskopische) Größenbestimmung **112** der Partikel des Wundmittel 10/Schüttguts 12 aus dem Wundmittel 10/Schüttgut 12 entfernt werden.

Das mechanische Zerkleinern erfolgt vorzugsweise bei einer geringen Luftfeuchte bzw. einer Feuchtigkeit der Umgebungsatmosphäre von unter 40%, insbesondere unter 20 %. In gleicher Weise wird das Wundmittel 10 vorzugsweise unter selbigen Bedingungen vakuumverpackt und so für den Einsatz am Menschen/Tier bzw. für eine Weiterverarbeitung bereitgestellt.

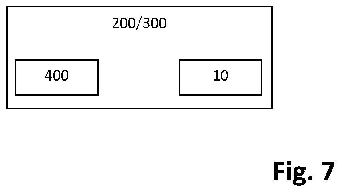

Das Wundmittel 10 kann gemäß **Fig. 7** auch Bestandteil eines Wundgels **200** eingesetzt werden. Das Wundgel 200 umfasst mit anderen Worten das (bevorzugt mikro-) partikuläre Wundmittel 10. Das Wundgel 200 kann darüber hinaus Wasser sowie ein wasserlösliches nicht-ionisches Polymer 400, insbesondere ein Cellulosederivat wie Hydroxypropylmethylcellulose, oder ein Polyglyceryl-2 Triisostearat als Emulgator sowie Filmbilder aufweisen. Das Wundgel eignet sich insbesondere für die intrakorporale, insbesondere intraabdominelle Applikation, etwa zur Adhäsionsprophylaxe nach abdominellen Eingriffen. Das Wundgel 200 kann selbst bei minimalinvasivem chirurgischem Zugang bequem im OP-Situs appliziert, insbesondere eingespritzt, werden. Das Wundgel 200 kann bei Bedarf zusätzlich einen Farbstoff aufweisen, anhand dessen die flächige Applikation vereinfacht visuell überprüft werden kann.

Das Wundmittel 10 kann gemäß Fig. 7 darüber hinaus als Füll- und Wirkstoff bei, insbesondere äußerlich anzuwendenden, Kosmetika 300 eingesetzt werden. Hier kann das Wundmittel 10 Hautreizungen sowie Mikroentzündungen der Haut als Vorstufe zu Akne vorbeugen und eine zügigere Regeneration der Haut begünstigen.

Darüber hinaus kann vorstehend erläuterte Schüttgut 12 auch als Trägersystem für Arzneistoffe dienen. Das Trägersystem kann aufgrund seiner Biokompatibilität und vollständigen Resorbierbarkeit eine verlangsamte Freisetzung des Arzneistoffs und damit einhergehend eine langfristige Wirkung des Arzneistoffs ermöglichen. Vorteilhaft ist, dass das Schüttgut 12 weder bei der extrakorporalen noch bei der endokorporalen Oberflächenanwendung toxisch ist und ohne Weiteres steril bereitgestellt werden kann.

## Patentansprüche

1. Wundmittel (10) in Form von Schuttgut (12) umfassend Partikel (14) mit einer maximalen Abmessung d von 5 mm, wobei die Partikel (14) jeweils eine Porosität von über 80% aufweisen und jeweils ein Co- und/oder Terpolymer auf der Basis von Polyhydroxycarbonsäuren, nämlich der Monomere Lactid, Trimethylencarbonat, Caprolacton und/oder 1,4-Dioxan-2-on oder Polyhydroxybutyrat (PHB) umfassen,
wobei die Partikel (14) eine bimodale Porenstruktur mit Porengröße im Bereich von 0,1 µm bis 50 µm, insbesondere im Bereich 0,5 bis 30 µm, und im Bereich von 80 µm bis 600 µm, insbesondere im Bereich von 100 µm bis 500 µm, aufweisen,
wobei die Partikel (14) mehrheitlich oder allesamt eine gespratzte Grundform aufweisen, sodass die Partikel nicht sphärisch oder gerundet ausgeführt sind,
wobei das Material des Wundmittels eine Glasübergangstemperatur zwischen 22°C und 45°C aufweist.

2. Wundmittel (10) gemäß Anspruch 1, **dadurch gekennzeichnet, dass** die Partikel (14) zumindest teilweise oder allesamt eine maximale Abmessung (d) von 2 mm, insbesondere 700 µm oder 150 µm, aufweisen.

3. Wundmittel (10) gemäß Anspruch 1 oder 2, **dadurch gekennzeichnet, dass** die Partikel (14) eine mittlere Porengröße im Bereich von 0,1 µm bis 50 µm, insbesondere im Bereich 0,5 bis 30 µm, aufweisen.

4. Wundmittel (10) gemäß Anspruch 2 oder 3, **dadurch gekennzeichnet, dass** die Partikel (14) eine Schüttdichte zwischen 0,08 g/ml und 0,16 g/ml, insbesondere zwischen 0,12 und 0,14 g/ml aufweisen.

5. Wundmittel (10) gemäß Anspruch 1, **dadurch gekennzeichnet, dass** die Partikel (14) eine maximale Abmessung d zwischen 1 mm und 5 mm und ein Volumengewicht zwischen 0,03 g/ml und 0,06 g/ml, insbesondere von ungefähr 0,04 g/ml aufweisen.

6. Wundmittel (10) gemäß einem der vorhergehenden Ansprüche, **dadurch gekennzeichnet, dass** die Partikel (14) jeweils einen Gehalt an freien Monomeren von 0 bis 12 Gew-%, insbesondere 0,1 bis 10 Gew.-%, vorzugsweise 1 bis 9 Gew.-%, bezogen auf das Gewicht des Co- und/oder Terpolymers, aufweisen.

7. Wundmittel (10) gemäß einem der vorhergehenden Ansprüche, **dadurch gekennzeichnet, dass** das Co- und/oder Terpolymer mit einem Füllmaterial, insbesondere einem synthetischen Polymer, vorzugsweise Polyvinylalkohol (PVA), dotiert ist.

8. Wundgel (200) oder Kosmetikum (300), **dadurch gekennzeichnet, dass** das Wundgel (200)/Kosmetikum (300) ein resorbierbares Wundmittel (10) gemäß einem der vorhergehenden Ansprüche umfasst.

9. Wundgel (200) oder Kosmetikum (300) gemäß Anspruch 8, **dadurch gekennzeichnet, dass** das Wundgel (200) /Kosmetikum (300) ein wasserlösliches nicht-ionisches Polymer (400), insbesondere ein Cellulosederivat, insbesondere Hydroxypropylmethylcellulose, und/oder ein Polyglyceryl-2 Triisostearat, umfasst.

## Claims

1. Wound agent (10) in the form of bulk material (12) comprising particles (14) with a maximum dimension d of 5 mm, wherein the particles (14) each have a porosity of more than 80% and each comprise a co- and/or terpolymer based on polyhydroxycarboxylic acids, namely the monomers lactide, trimethylene carbonate, caprolactone and/or 1,4-dioxan-2-one or polyhydroxybutyrate (PHB),
wherein the particles (14) have a bimodal pore structure with a pore size in the range from 0.1 µm to 50 µm, in particular in the range from 0.5 to 30 µm, and in the range from 80 µm to 600 µm, in particular in the range from 100 µm to 500 µm,
wherein the majority or all of the particles (14) have a spattered basic shape, so that the particles are not spherical or rounded,
wherein the material of the wound agent has a glass transition temperature between 22°C and 45°C.

2. Wound agent (10) according to claim 1, **characterized in that** the particles (14) at least partially or all have a maximum dimension (d) of 2 mm, in particular 700 µm or 150 µm.

3. Wound agent (10) according to either claim 1 or claim 2, **characterized in that** the particles (14) have an average pore size in the range of 0.1 µm to 50 µm, in particular in the range of 0.5 to 30 µm.

4. Wound agent (10) according to either claim 2 or claim 3, **characterized in that** the particles (14) have a bulk density between 0.08 g/ml and 0.16 g/ml, in particular between 0.12 and 0.14 g/ml.

5. Wound agent (10) according to claim 1, **characterized in that** the particles (14) have a maximum dimension d between 1 mm and 5 mm and a volume weight between 0.03 g/ml and 0.06 g/ml, in particular of approximately 0.04 g/ml.

6. Wound agent (10) according to any of the preceding claims, **characterized in that** the particles (14) each have a content of free monomers of 0 to 12 wt.%, in particular 0.1 to 10 wt.%, preferably 1 to 9 wt.%, based on the weight of the co- and/or terpolymer.

7. Wound agent (10) according to any of the preceding claims, **characterized in that** the co- and/or terpolymer is doped with a filler material, in particular a synthetic polymer, preferably polyvinyl alcohol (PVA).

8. Wound gel (200) or cosmetic (300), **characterized in that** the wound gel (200)/cosmetic (300) comprises a resorbable wound agent (10) according to any of the preceding claims.

9. Wound gel (200) or cosmetic (300) according to claim 8, **characterized in that** the wound gel (200)/cosmetic (300) comprises a water-soluble non-ionic polymer (400), in particular a cellulose derivative, in particular hydroxypropylmethylcellulose, and/or a polyglyceryl-2 triisostearate.

## Revendications

1. Agent cicatrisant (10) sous forme de produit en vrac (12) comprenant des particules (14) d'une dimension maximale d de 5 mm, les particules (14) présentant respectivement une porosité supérieure à 80 % et comprenant respectivement un copolymère et/ou un terpolymère à base d'acides polyhydroxycarboxyliques, à savoir les monomères lactide, carbonate de triméthylène, caprolactone et/ou 1,4-dioxan-2-one ou polyhydroxybutyrate (PHB),
les particules (14) présentant une structure poreuse bimodale avec une taille de pore dans la plage comprise entre 0,1 µm et 50 µm, en particulier dans la plage comprise entre 0,5 µm et 30 µm, et dans la plage comprise entre 80 µm et 600 µm, en particulier dans la plage comprise entre 100 µm et 500 µm,
les particules (14) présentant majoritairement ou en totalité une forme de base éclatée, de sorte que les particules ne sont pas sphériques ou arrondies,
le matériau de l'agent cicatrisant présentant une température de transition vitreuse comprise entre 22°C et 45°C.

2. Agent cicatrisant (10) selon la revendication 1, **caractérisé en ce que** les particules (14) présentent au moins partiellement ou en totalité une dimension maximale (d) de 2 mm, en particulier 700 µm ou 150 µm.

3. Agent cicatrisant (10) selon la revendication 1 ou 2, **caractérisé en ce que** les particules (14) présentent une taille de pore moyenne dans la plage comprise entre 0,1 µm et 50 µm, en particulier dans la plage comprise entre 0,5 µm et 30 µm.

4. Agent cicatrisant (10) selon la revendication 2 ou 3, **caractérisé en ce que** les particules (14) présentent une densité apparente comprise entre 0,08 g/ml et 0,16 g/ml, en particulier comprise entre 0,12 g/ml et 0,14 g/ml.

5. Agent cicatrisant (10) selon la revendication 1, **caractérisé en ce que** les particules (14) présentent une dimension maximale d comprise entre 1 mm et 5 mm et un poids volumique compris entre 0,03 g/ml et 0,06 g/ml, en particulier d'environ 0,04 g/ml.

6. Agent cicatrisant (10) selon l'une quelconque des revendications précédentes, **caractérisé en ce que** les particules (14) présentent respectivement une teneur en monomères libres comprise entre 0 et 12 % en poids, en particulier comprise entre 0,1 et 10 % en poids, de manière préférée comprise entre 1 et 9 % en poids, par rapport au poids du copolymère et/ou du terpolymère.

7. Agent cicatrisant (10) selon l'une quelconque des revendications précédentes, **caractérisé en ce que** le copolymère et/ou le terpolymère est/sont dopé(s) avec un matériau de charge, en particulier un polymère synthétique, de manière préférée de l'alcool polyvinylique (PVA).

8. Gel cicatrisant (200) ou produit cosmétique (300), **caractérisé en ce que** le gel cicatrisant (200)/le produit cosmétique (300) comprend un agent cicatrisant (10) résorbable selon l'une quelconque des revendications précédentes.

9. Gel cicatrisant (200) ou produit cosmétique (300) selon la revendication 8, **caractérisé en ce que** le gel cicatrisant (200)/le produit cosmétique (300) comprend un polymère non ionique (400) soluble dans l'eau, en particulier un dérivé de cellulose, en particulier de l'hydroxypropylméthylcellulose, et/ou du polyglycéryl-2 triisostéarate.
